# FASCICULE DE BREVET EUROPEEN

(11) **EP 1 603 627 B1**
(45) Date de publication et mention de la délivrance du brevet: **19.05.2010**
(21) Numéro de dépôt: 04718672.1
(22) Date de dépôt: 09.03.2004
(51) Int. Cl.: A61M 25/02

(54) **DISPOSITIF DE FIXATION AU CORPS D'UN PATIENT D'UN CATHETER**
VORRICHTUNG ZUR FIXATION EINES KATHETERS AM KÖRPER EINES PATIENTEN
DEVICE FOR FIXING A CATHETER TO THE BODY OF A PATIENT

(30) Priorité: 19.03.2003 FR 0303350
(43) Date de publication de la demande: 14.12.2005
(73) Titulaire: Navarro, Francis, 34000 Montpellier (FR); Le Bozec, Jacques, 35500 La Chapelle Erbree (FR)
(72) Inventeur: Navarro, Francis, 34000 Montpellier (FR); Le Bozec, Jacques, 35500 La Chapelle Erbree (FR)
(74) Mandataire: Thinat, Michel
(86) Numéro de dépôt international: PCT/FR2004/000560
(87) Numéro de publication internationale: WO 2004/087250

(56) Documents cités:
- EP-A- 0 286 525
- EP-A- 0 931 560
- FR-A- 2 787 336
- US-A- 4 517 971

## Description

La présente invention concerne un dispositif de fixation au corps d'un patient d'un cathéter, tel qu'un cathéter veineux périphérique, un cathéter veineux central ou un cathéter artériel central, ou d'une aiguille de ponction, telle qu'une aiguille d'Huber coudée à angle droit.

L'administration de produits médicamenteux dans le système circulatoire du corps humain s'effectue soit par voie veineuse, soit par voie artérielle..

Pour administrer ces produits médicamenteux, il est nécessaire de mettre en place un cathéter constitué par un tuyau de petit diamètre inférieur à celui des vaisseaux qu'ils soient veineux ou artériels. De tels cathéters peuvent être utilisés non seulement pour injecter des produits médicamenteux, mais également pour capter des pressions sanguines soit dans le système veineux, soit dans le système artériel.

D'une manière générale, on distingue ainsi deux types de cathéters, les cathéters de voies veineuses périphériques et les cathéters de voies veineuses centrales ou de voies artérielles.

Les cathéters veineux périphériques sont posés au niveau d'une veine périphérique, c'est-à-dire habituellement au niveau des membres supérieurs du patient, la veine considérée pouvant être une veine de l'avant-bras ou une veine du pli du coude. Parmi ces cathéters, on connaît le cathéter veineux unique relativement court, d'environ 5 à 6 centimètres de longueur, posé dans une veine périphérique et ayant sa partie sortant de la veine fixée à la peau du patient par de simples adhésifs, pour administrer des produits ou solutions pendant quelques heures, c'est-à-dire pendant une période de temps relativement limitée. Le point de ponction peut être changé à plusieurs reprises en fonction de la résistance des veines périphériques.

Le cathéter veineux central ou le cathéter artériel central est mis en place dans des axes veineux profonds, c'est-à-dire les grosses veines. Il s'agit habituellement de la veine jugulaire interne ou externe, de la veine sous-clavière ou de la veine fémorale.

La mise en place de ces cathéters s'effectue par voie cutanée, c'est-à-dire en regard de la peau et dans la zone où se trouve la veine dans laquelle on souhaite poser le cathéter. Pour cela, on effectue une ponction cutanée jusque dans la veine par l'intermédiaire d'un trocart rigide, qui est une sorte de tuteur, muni d'une seringue à son extrémité opposée à sa partie introduite dans la veine permettant l'aspiration du sang veineux et une fois ce trocart positionné dans l'axe veineux, et le cathéter est introduit après retrait de la seringue pour permettre la mise en place de ce cathéter dans la veine de plus gros diamètre.

En ce qui concerne le cathéter de voie veineuse centrale ou le cathéter artériel central, celui-ci est relié à son extrémité opposée à sa partie introduite dans la grosse veine, c'est-à-dire immédiatement à sa sortie au niveau de la peau, à un support, généralement en matière plastique rigide, constituant une partie de jonction de ce cathéter à un ou plusieurs cathéters ou tuyaux externes d'un nombre pouvant aller jusqu'à cinq et dans lesquels peuvent circuler plusieurs produits différents pouvant être administrés en même temps et arrivant dans le support en forme de boîtier puis introduits dans le cathéter posé dans la grosse veine. Ce support est habituellement de forme générale triangulaire, parfois circulaire, et est muni de deux ailettes externes permettant de fixer le support à la peau du patient par l'intermédiaire d'un fil de suture.

Cependant, les moyens de fixation à la peau de ces différents cathéters ont pour inconvénient majeur d'être le siège d'infections au niveau du site de ponction et de pénétration du cathéter veineux ou artériel et, par conséquent, il est nécessaire d'effectuer des pansements quotidiens pour éviter de telles infections pouvant aller jusqu'à provoquer une septicémie. En outre, lorsque ces pansements sont mis en place au niveau fémoral ou jugulaire, ils entraînent un réel encombrement de la zone anatomique avec une mobilité diminuée et difficile pour le patient porteur d'un cathéter veineux central.

De plus, comme déjà mentionné précédemment, les cathéters de voie veineuses centrales ont pour inconvénient d'utiliser des fils de suture pour fixer le support de ce cathéter auquel arrivent les cathéters externes avec les risques que cela incombe aux opérateurs, à savoir les réanimateurs et les infirmières.

En outre, l'administration de produits de chimiothérapie est également effectuée par voie veineuse centrale, c'est-à-dire par la pose d'un cathéter dans une grosse veine et ce cathéter est relié à une chambre d'injection implantée sous la peau généralement au niveau pectoral droit ou gauche du patient. L'accès s'effectue au niveau de l'artère jugulaire interne et la chambre sous-cutanée est ponctionnée par une aiguille d'Huber coudée à angle droit qui est laissée en place le temps de la perfusion afin que le produit chimiothérapique soit administré dans de bonnes conditions. La mise en place de cette aiguille nécessite son maintien sur la peau par des moyens adhésifs, qui non seulement sont inadaptés, mais peuvent également entraîner dans certaines circonstances des infections au point de ponction cutanée. Par conséquent, il est nécessaire d'utiliser des pansements et des compresses de protection pour éviter toute infection et permettre la chimiothérapie.

La présente invention a pour but d'éliminer les inconvénients ci-dessus en proposant un dispositif permettant la protection de la zone de ponction cutanée qui est souvent le siège d'infections, de contrôler le point de ponction et d'éviter l'utilisation des pansements quotidiens pour le nettoyage des plaies.

A cet effet, l'invention propose un dispositif de fixation selon la revendication 1.

Lorsque le dispositif est utilisé pour la fixation d'un cathéter, dont l'embase constitue un petit réservoir de raccordement au tuyau externe, le boîtier est plat et fait saillie de son embase suivant une hauteur relativement faible, et en ce que les première et seconde chambres sont situées approximativement dans un même plan et le couvercle comprend à sa face interne deux bourrelets pénétrant dans la seconde chambre en position fermée du couvercle de manière à venir en appui respectivement sur deux faces latérales de l'embase du cathéter pour la maintenir bilatéralement relativement au fond plat de la seconde chambre également fixé sur la peau du patient.

Les bourrelets viennent en appui, en position fermée du couvercle, respectivement sur deux ailettes s'étendant de part et d'autre des faces latérales de l'embase du cathéter pour maintenir cette dernière en appui sur le fond de la seconde chambre.

Les deux chambres sont séparées l'une de l'autre par une paroi, dont la face supérieure est située dans le plan de la face supérieure du boîtier, et comportant un passage de communication défini par deux faces latérales obliques perpendiculaires au fond de la seconde chambre et convergeant vers la première chambre et en ce que l'embase du cathéter a ses faces latérales en prolongement de celles retenues par les bourrelets de forme conjuguée aux faces latérales du passage pour permettre à l'embase du cathéter de s'engager en partie dans le passage et y être retenue.

La première chambre a son fond constitué par une membrane souple relativement mince fixée sur la peau du patient et comportant un orifice permettant le passage de l'embase du cathéter et de ce cathéter.

La membrane comporte des fentes partant du bord délimitant l'orifice de passage précité.

Selon une variante de réalisation, le couvercle comprend à sa face interne deux autres bourrelets pénétrant dans la première chambre en position fermée du couvercle de manière à être disposés de part et d'autre de la partie externe du cathéter posé dans la veine en obturant en grande partie cette chambre et à venir en appui sur la peau du patient.

Chaque bourrelet comprend sur sa face en contact avec la peau un colloïde pouvant contenir des substances antiseptiques ou antimicrobiennes.

Le boîtier comprend sur sa face supérieure délimitant la seconde chambre, à l'opposé de la communication entre les deux chambres, au moins une rainure longitudinale de réception du tuyau externe raccordé à l'embase de jonction avec le cathéter et la face interne du couvercle comprend également au moins une rainure longitudinale venant au-dessus de la rainure du boîtier en position fermée du couvercle pour maintenir le tuyau relativement au boîtier, le tuyau sortant de ce boîtier pouvant être raccordé à des moyens de perfusion, de transfusion ou de prélèvements sanguins.

Pour la fixation d'un cathéter artériel central, la seconde chambre débouche directement dans la première chambre et est constituée par une partie évidée de forme coopérant avec une partie évidée identique définie entre les deux bourrelets du couvercle de manière à former en position fermée du couvercle un logement conjugué à l'embase du cathéter artériel central permettant de retenir cette embase dans le boîtier.

L'embase de fixation du boîtier est constituée par une nappe en matériau souple venant de moulage avec le boîtier et, le cas échéant, la membrane de la première chambre, les faces du fond de la seconde chambre et de la membrane en appui sur la peau du patient étant continues avec la face d'appui de l'embase sur la peau.

L'embase de fixation du boîtier comprend au moins deux pattes d'appui chacune en forme d'oreille.

L'embase comprend quatre pattes d'appui en forme d'oreilles.

Le couvercle est monté articulé au boîtier et peut être verrouillé à ce dernier par encliquetage.

L'embase du boîtier est fixée à la peau du patient par un colloïde pouvant contenir des substances antiseptiques ou antimicrobiennes.

L'invention sera mieux comprise, et d'autres buts, caractéristiques, détails et avantages de celle-ci apparaîtront plus clairement dans la description explicative qui va suivre faite en référence aux dessins schématiques annexés donnés uniquement à titre d'exemple illustrant plusieurs modes de réalisation de l'invention et dans lesquels :
- la figure 1 est une vue en perspective du dispositif de fixation au corps d'un patient d'un cathéter suivant un premier mode de réalisation de l'invention ;
- la figure 2 est une vue en perspective suivant un angle différent du dispositif de la figure 1, sans le cathéter ;
- la figure 3 est une vue en perspective du dispositif de la figure 1 en position semi-fermée du couvercle du boîtier de ce dispositif ;
- la figure 4 est une vue de dessous du dispositif des figures 1 à 3 ;
- la figure 5 représente un second mode de réalisation du dispositif de fixation de l'invention ;
- la figure 6 est une vue en perspective d'un troisième mode de réalisation du dispositif de l'invention ;
- la figure 7 est une vue en perspective du dispositif de la figure 6 dont le boîtier est fermé par son couvercle.

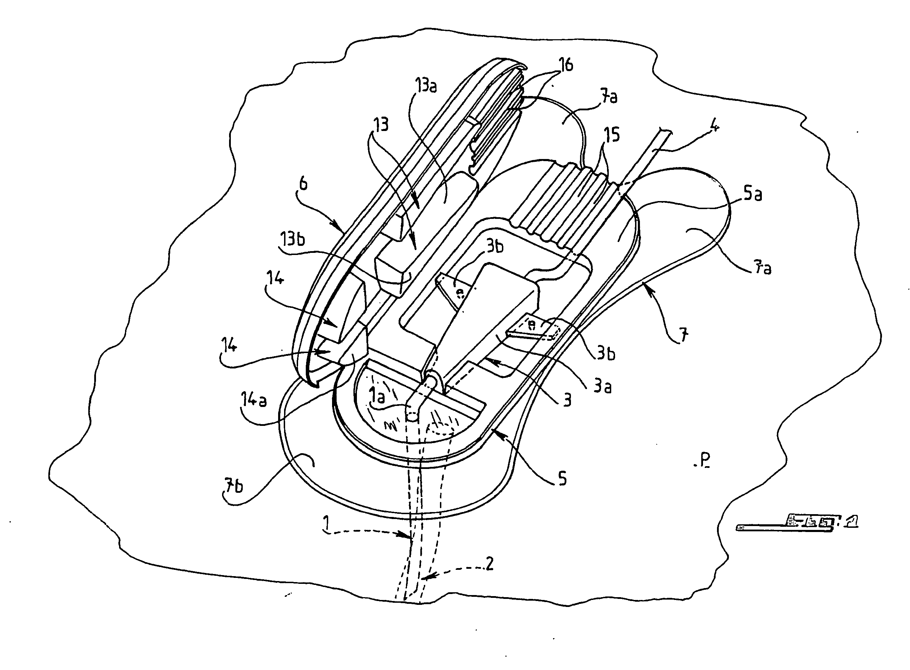

Le dispositif représenté aux figures 1 à 4 s'applique à la fixation au corps d'un patient d'un cathéter veineux central 1 posé suivant la technique mentionnée précédemment dans une grosse veine 2 du corps du patient par voie sous-cutanée, le cathéter 1 ayant sa partie externe la sortant de la peau P raccordée à une embase de support 3 constituant un petit réservoir fermé et étanche raccordé à son extrémité opposée au cathéter 1 à au moins un cathéter ou tuyau externe 4 en communication du fluide avec le cathéter 1 pour administrer dans le réservoir 1 un produit médicamenteux. Un à cinq cathéters externes 4 peuvent être raccordés au réservoir 1 pour administrer plusieurs produits médicamenteux différents dans la veine 2 au travers du réservoir 3 et du cathéter veineux 1. Bien entendu, chaque cathéter externe 4 est raccordé à des moyens de perfusion connus en soi.

Selon l'invention, le dispositif comprend un boîtier 5 pouvant être fermé par un couvercle 6 monté articulé sur un côté du boîtier qui est plat et, par conséquent, de relativement faible épaisseur, par exemple d'environ 8 à 10 mm.

Le dispositif comprend en outre une embase 7 solidaire du boîtier 5 en entourant celui-ci pour permettre la fixation du boîtier sur la peau P du patient notamment par un colloïde.

Le boîtier 5 comprend deux chambres communiquant l'une avec l'autre, une première chambre 8 traversant le fond du boîtier est située au-dessus du site de ponction du cathéter 1 en étant traversée par la partie externe la de ce cathéter et une seconde chambre 9 dans laquelle est logé le réservoir 3 de jonction entre le cathéter externe 4 et le cathéter veineux 1.

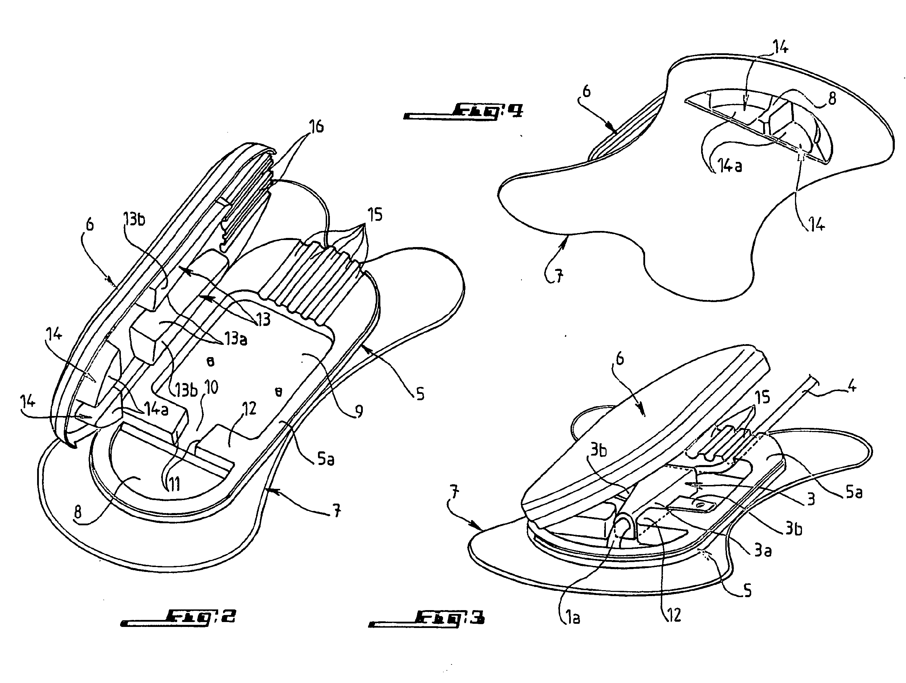

Comme cela ressort de la figure 2, la chambre 8 peut présenter, vue de dessus, une forme semi-circulaire tandis que la chambre 9 peut présenter vu de dessus, une forme rectangulaire.

Compte tenu de la relativement faible épaisseur du boîtier 5, les deux chambres 8 et 9 sont approximativement dans le même plan et communiquent l'une avec l'autre par l'intermédiaire d'un passage 10 délimité par deux parois latérales 11 solidaires du fond de la chambre 9 perpendiculairement à celui-ci et inclinées l'une relativement à l'autre en convergeant vers la chambre 8. Le passage 10 est réalisé au travers d'une paroi 12 solidaire de la partie de fond de la chambre 9 adjacente à la chambre 8 et dont la face supérieure plane fait partie de la face supérieure plane 5a du boîtier 5.

Le réservoir 3 est de forme prismatique à section transversale sensiblement rectangulaire et dont les parois latérales 3a disposées perpendiculairement au fond de la chambre 9 lorsque le réservoir 3 est logé dans celle-ci, sont inclinées l'une par rapport à l'autre et convergent l'une vers l'autre vers la paroi en bout de raccordement du réservoir 3 à la partie externe la du cathéter 1.

Le réservoir 3 peut comporter deux ailettes 3b solidaires respectivement des parois latérales 3a symétriquement au plan longitudinal du réservoir 3.

Lorsque le réservoir 3 est logé dans la chambre 9 du boîtier 5, la partie la plus étroite de ce réservoir formant nez est logée dans le passage 10 avec les parties correspondantes des parois latérales obliques 3a en appui respectivement sur les deux parois latérales 11 de ce passage. De la sorte, le réservoir 3 est maintenu dans le passage 10 et ne peut se déplacer vers la chambre 8.

Le couvercle 6 comprend solidaire de sa face interne deux bourrelets prismatiques longilignes 13 disposés symétriquement au plan médian longitudinal du couvercle 6 et dont les faces latérales en regard l'une de l'autre 13a s'étendant perpendiculairement à la face interne du couvercle 6, sont disposées obliquement l'une par rapport à l'autre de la même manière que les parois latérales 3a du réservoir 3. En position de fermeture du couvercle 6, les deux bourrelets 13 pénètrent dans la chambre 9 et viennent sensiblement en appui par leurs faces obliques 13a respectivement avec les parois obliques 3a du réservoir 3 de manière à maintenir ce dernier bilatéralement relativement au boîtier 5. En outre, les bourrelets 13 viennent en appui par leurs faces planes terminales 13b respectivement sur les deux ailettes 3b du réservoir 3. De la sorte, le réservoir 3, en position de fermeture du couvercle 6, est parfaitement immobilisé dans la chambre 9, ce qui empêche tout retrait accidentel de ce réservoir et, par conséquent, du cathéter veineux 1, éliminant tout risque d'hémorragie importante. Les ailettes 3b peuvent être positionnées au fond de la chambre 9 par deux pions 9a solidaires de ce fond et s'engageant dans deux perçages des ailettes 3b.

Le couvercle 6 comprend deux autres bourrelets 14 solidaires de sa face interne et pénétrant dans la chambre 8 en position de fermeture du couvercle 6 de manière à obturer partiellement cette dernière. Les deux bourrelets 14 sont espacés l'un de l'autre de manière à se trouver sensiblement en prolongement du passage 10 en position de fermeture du couvercle 6 et à être disposés de part et d'autre de la partie externe 1a du cathéter veineux 1. En outre, les faces planes terminales 14a des bourrelets 14 viennent en appui sur la peau en position de fermeture du couvercle 6 et, de préférence, ces faces terminales 14a peuvent comporter un colloïde ou servir de support à un produit antiseptique ou antimicrobien. Bien entendu, les parois périphériques externes des bourrelets 14 sont de forme conjuguée aux parois périphériques délimitant la chambre 8 de manière à être pratiquement jointive avec ces dernières en position de fermeture du couvercle 6.

Le boîtier 5 comporte sur sa face supérieure 5a opposée à la paroi 12 plusieurs rainures longitudinales 15, dans le cas présent au nombre de cinq, chacune à section transversale semi-circulaire et pouvant recevoir un cathéter externe 4. Le couvercle 6 comporte également des rainures longitudinales identiques 16 réalisées dans sa face interne et venant au-dessus respectivement des rainures longitudinales 15 du boîtier 5 en position de fermeture du couvercle 6 de manière à maintenir entre elles respectivement les différents cathéters externes 4 raccordés au réservoir 3.

Le couvercle 6 peut être verrouillé à sa position de fermeture du boîtier 5 par des moyens d'encliquetage, non représentés, et pouvant être constitués par une partie semi-cylindrique du bord longitudinal libre du couvercle 6 opposée à son articulation et une rainure conjuguée du bord externe supérieur du boîtier 5 recevant la partie semi-cylindrique.

L'embase 7 de fixation du boîtier 5 est constituée par une nappe en matériau souple venant de matière, par exemple par moulage, avec le boîtier 5 de manière que la face inférieure d'appui de cet ensemble sur la peau du patient soit plane et pleine sauf à l'endroit où débouche la chambre 8 et puisse épouser la forme anatomique du patient sur laquelle doit être fixé le dispositif.

Le couvercle 6 peut être réalisé en une seule pièce par moulage et, comme le boîtier 5 et l'embase 7, peut être en une matière plastique.

Selon ce premier mode de réalisation, l'embase 7 est conformée en vue de dessus, comme comprenant deux pattes d'appui 7a, chacune en forme d'oreille, situées à l'opposée de la chambre 8 et une partie arquée opposée 7b adjacente à la chambre 8. Le couvercle 6 et par conséquent le boîtier 5 présente en vue de dessus une forme rectangulaire à extrémités arquées.

La mise en place du dispositif ci-dessus décrit s'effectue comme suit. Après avoir posé le cathéter 1 dans la grosse veine, le réservoir 3 et la partie externe 1a du cathéter 1 sont introduits au travers de la chambre 8 du boîtier 5 et le réservoir 3 est encastré dans le passage 10 en appui sur le fond de la chambre 9. Ensuite, le ou les cathéters externes 4 sont raccordés au réservoir 3 et disposés dans leurs rainures respectives 15, à moins qu'ils soient déjà livrés raccordés à celui-ci, auquel cas le ou les cathéters 4 sont préalablement introduits au travers de la fenêtre 8 avant d'être disposés dans leurs rainures respectives 15 du boîtier 5. Ensuite, l'opérateur rabat le couvercle 6 pour l'amener à sa position de fermeture à laquelle les bourrelets 13 immobilisent complètement le réservoir 3 dans la chambre 9 et les cathéters externes 4 relativement au boîtier 5, et l'embase 7, recouverte sur sa face interne de colloïde, est fixée sur la peau du patient. Le colloïde appliqué sur la face interne de l'embase 7 peut être protégé par un film approprié, non représenté, qui est retiré avant d'appliquer le dispositif sur la peau du patient. Le colloïde peut contenir des substances antiseptiques de façon à diminuer considérablement les risques d'infections.

Afin de visualiser la zone de ponction du cathéter 1, le capot 6 peut être réalisé en une matière plastique transparente.

Le dispositif ci-dessus décrit empêche ainsi toute extraction accidentelle du réservoir 3 du cathéter veineux 1 et peut parfaitement s'adapter à la zone anatomique du patient, ce qui procure au patient un confort et lui permet de se déplacer plus aisément. Le dispositif évite toute fixation à la peau du support ou embase 3 en forme de réservoir par des fils de suture.

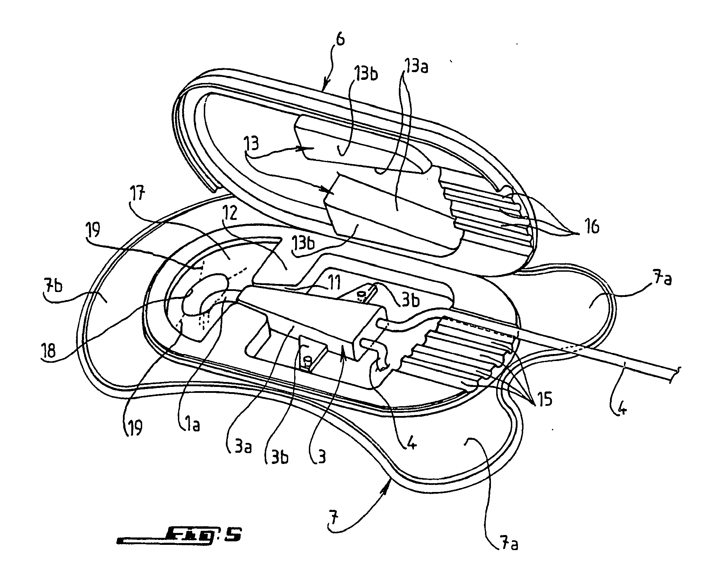

Le second mode de réalisation du dispositif de l'invention représenté en figure 5 ne diffère du dispositif du premier mode de réalisation que par l'absence des deux bourrelets 14 et la présence d'une membrane souple relativement mince 17 constituant le fond de la chambre 8 et comportant un orifice 18 permettant le passage du réservoir 3 avec ses ailettes 3b et de la partie externe la du cathéter veineux 1 une fois ce dernier posé. La membrane 17 peut comporter des fentes 19 partant du bord de l'orifice 18 pour permettre le passage du réservoir 3 et de ses ailettes au travers de l'orifice 18 par déformation élastique des languettes définies entre les différentes fentes 19 qui s'étendent radialement dans le cas d'un orifice 18 circulaire. En outre, la face interne de la membrane 19 située dans le même plan que la face interne de l'embase 7 et qui peut être réalisée par moulage avec cette dernière et le boîtier 5, peut être revêtue d'un colloïde permettant d'accoler la membrane 17 à la peau du patient et le colloïde peut comporter des substances antiseptiques ou antimicrobiennes.

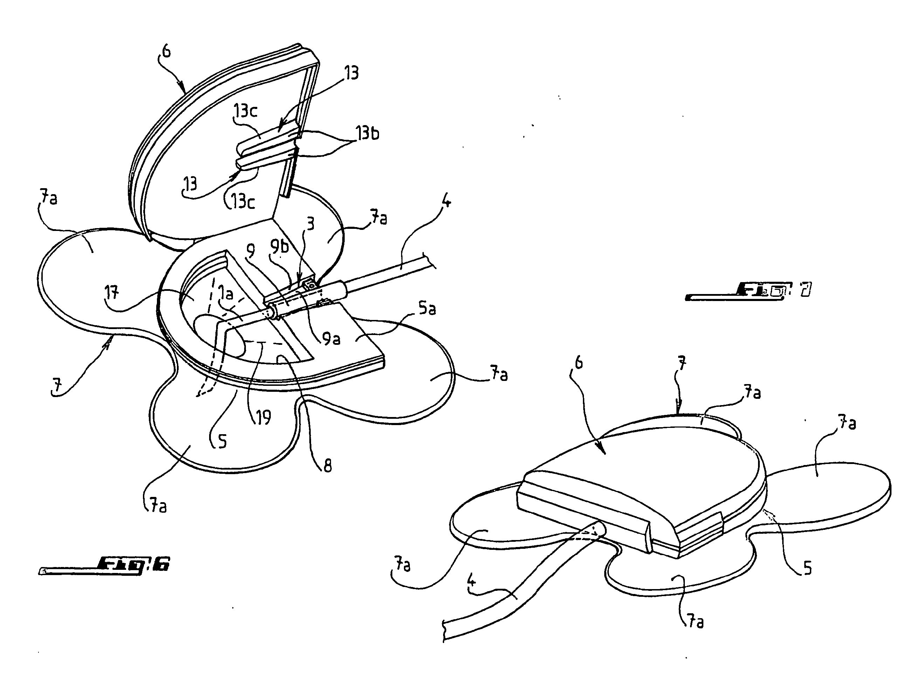

Selon le troisième mode de réalisation des figures 6 et 7, le boîtier 5 est plat comme dans les modes de réalisation précédents et, en vue de dessus, présente une forme en demi-cercle prolongée par deux côtés parallèles raccordés par un côté transversal définissant la paroi arrière du boîtier 5 opposée à la paroi avant arquée et la seconde chambre 9, qui débouche directement dans la chambre 8, est définie par une partie évidée longiligne à section transversale semi-circulaire formée dans la paroi supérieure 5a du boîtier 5 en s'étendant perpendiculairement à la paroi latérale droite de la chambre 8 de forme semi-circulaire lorsque vue de dessus. La partie évidée 9 peut s'évaser à partir de son extrémité débouchant dans la chambre 8 afin de recevoir l'embase en forme de manchon 3 du cathéter 1 dans le cas présent du type veineux périphérique ou artérielle à voie unique, c'est-à-dire qu'un seul cathéter externe est raccordé au manchon 3. Ce dernier peut avoir une forme générale effilée tronconique de forme conjuguée à la chambre de réception 9. Les deux bourrelets 13 de la face interne du couvercle 6 définissent entre eux un espace de forme conjuguée à la partie supérieure du manchon de cathéter 9 de manière à emprisonner le manchon 3 dans le boîtier 5 en position fermée du couvercle 6. L'évidement définissant la chambre 9 est délimité en partie supérieure par deux faces planes latérales 9a parallèles à la face supérieure 5a du boîtier 5 et raccordées à cette dernière par deux épaulements 9b s'étendant obliquement en divergeant à l'opposé de la chambre 8 dans le cas où le manchon 3 est tronconique. En position fermée du couvercle 6, les deux bourrelets 13 sont simplement en appui par leurs faces d'extrémités 13b et leurs faces latérales externe 13c respectivement sur les faces 9a et les épaulements 9b.

L'embase 7 de fixation du boîtier 5 est définie par quatre parties en forme d'oreille 7a disposées symétriquement au plan médian longitudinal du dispositif. Ce dernier peut comporter une membrane souple 17 constituant le fond de la chambre 8 comme dans le second mode de réalisation.

## Revendications

1. Dispositif de fixation au corps d'un patient d'un cathéter (1), tel qu'un cathéter veineux périphérique, un cathéter veineux central ou un cathéter artériel central, comprenant un boîtier (5) pouvant être fermé par un couvercle (6), une embase (7) solidaire du boîtier (5) en entourant celui-ci et permettant de fixer le boîtier (5) sur la peau du patient, le boîtier (5) comprenant deux chambres (8,9) communicant l'une avec l'autre, une première chambre (8) située au-dessus du site de ponction du cathéter et adaptée à être traversée par la partie externe (1a) du cathéter (1) posé dans la veine ou l'artère (2), et une seconde chambre (9) permettant le logement d'une embase (3) raccordée à l'une de ses extrémités au cathéter, afin que laquelle embase (3) soit retenue dans la seconde chambre et raccordée à son extrémité opposée à au moins un tuyau externe (4) en communication de fluide avec le cathéter au travers de l'embase (3) , et en ce que le boîtier (5) est plat et fait saillie de son embase (7) suivant une hauteur relativement faible, et en ce que les première et seconde chambres (8,9) sont situées approximativement dans un même plan, **caractérisé en ce que** le couvercle (6) comprend à sa face interne deux bourrelets (13) pénétrant dans la seconde chambre (9) en position fermée du couvercle (6) de manière à venir en appui respectivement sur deux faces latérales (3a) de l'embase (3) du cathéter (1) pour la maintenir bilatéralement relativement au fond plat de la seconde chambre (9) également fixé sur la peau du patient.

2. Dispositif selon la revendication 1, **caractérisé en ce que** les deux bourrelets (13) sont adaptés à venir en appui, en position fermée du couvercle (6), respectivement sur deux ailettes (3b) s'étendant de part et d'autre des faces latérales (3a) de l'embase (3) du cathéter (1) pour maintenir cette dernière en appui sur le fond de la seconde chambre (9).

3. Dispositif selon la revendication 1 ou 2, **caractérisé en ce que** les deux chambres (8,9) sont séparées l'une de l'autre par une paroi (12), dont la face supérieure est située dans le plan de la face supérieure (5a) du boîtier (5), et comportant un passage de communication (10) défini par deux faces latérales obliques (11) perpendiculaires au fond de la seconde chambre (9) et convergeant vers la première chambre (8) et **en ce que** l'embase (3) du cathéter (1) a ses faces latérales en prolongement de celles retenues par les bourrelets (13) de forme conjuguée aux faces latérales (11) du passage (10) pour permettre à l'embase (3) du cathéter (1) de s'engager en partie dans le passage (10) et y être retenue.

4. Dispositif selon l'une des revendications 1 à 3, **caractérisé en ce que** la première chambre (8) a son fond constitué par une membrane souple relativement mince (17) adaptée à être fixée sur la peau du patient et comportant un orifice (18) permettant le passage de l'embase du cathéter (3) et de ce cathéter.

5. Dispositif selon la revendication 4, **caractérisé en ce que** la membrane (17) comporte des fentes (19) partant du bord délimitant l'orifice de passage (18) précité.

6. Dispositif selon l'une des revendications 1 à 3, **caractérisé en ce que** le couvercle (6) comprend à sa face interne deux autres bourrelets (14) pénétrant dans la première chambre (8) en position fermée du couvercle (6) de manière à être disposés de part et d'autre de la partie externe (1a) du cathéter (1) posé dans la veine (2) en obturant en grande partie cette chambre et à venir en appui sur la peau du patient.

7. Dispositif selon la revendication 6, **caractérisé en ce que** chaque bourrelet (14) comprend sur sa face (14a) destinée à venir en contact avec la peau un colloïde pouvant contenir une substance antiseptique ou antimicrobienne.

8. Dispositif selon l'une des revendications 1 à 7, **caractérisé en ce que** le boîtier (5) comprend sur sa face supérieure (5a) délimitant la seconde chambre (9), à l'opposé de la communication (10) entre les deux chambres, (8,9) au moins une rainure longitudinale (15) de réception du tuyau externe (4) raccordé à l'embase (3) de jonction avec le cathéter (1) et la face interne du couvercle (6) comprend également au moins une rainure longitudinale (16) venant au-dessus de la rainure (15) du boîtier (5) en position fermée du couvercle (6) pour maintenir le tuyau (4) relativement au boîtier (5), le tuyau (4) sortant de ce boîtier pouvant être raccordé à des moyens de perfusion, de transfusion ou de prélèvements sanguins.

9. Dispositif selon l'une des revendications 1, 4 à 7, **caractérisé en ce que** la seconde chambre (9) débouche directement dans la première chambre (8) et est constituée par une partie évidée de forme coopérant avec une partie évidée identique définie entre les deux bourrelets (13) du couvercle (6) de manière à former en position fermée du couvercle (6) un logement conjugué à l'embase (3) du cathéter artériel central (1) permettant de retenir cette embase dans le boîtier (5).

10. Dispositif selon l'une des revendications précédentes, **caractérisé en ce que** l'embase (7) de fixation du boîtier (5) est constituée par une nappe en matériau souple venant de moulage avec le boîtier (5) et, le cas échéant, la membrane (17) de la première chambre (8), les faces du fond de la seconde chambre (8) et de la membrane (17) en appui sur la peau du patient étant continues avec la face d'appui de l'embase (7) sur la peau.

11. Dispositif selon la revendication 10, **caractérisé en ce que** l'embase (7) de fixation du boîtier (5) comprend au moins deux pattes d'appui (7a) chacune en forme d'oreille.

12. Dispositif selon la revendication 11, **caractérisé en ce que** l'embase (7) comprend quatre pattes d'appui (7a) en forme d'oreilles.

13. Dispositif selon l'une des revendications précédentes, **caractérisé en ce que** le couvercle (6) est monté articulé au boîtier (5) et peut être verrouillé à ce dernier par encliquetage.

14. Dispositif selon l'une des revendications précédentes, **caractérisé en ce que** l'embase (7) du boîtier (5) est adaptée à être fixée à la peau du patient par un colloide pouvant contenir des substances antiseptiques ou antimicrobiennes.

## Claims

1. A device for fixing to the body of a patient a catheter (1), such as a peripheral venous catheter, a central venous catheter or a central arterial catheter, comprising a case (5), which can be closed by a lid (6), a base (7) integral with the case (5) by surrounding the same, and allowing for the case (5) to be fixed to the patient's skin, the case (5) comprising two chambers (8, 9) communicating with each other, a first chamber (8) located above the puncture site of the catheter and suitable for being traversed by the external part (1a) of the catheter (1) placed inside the vein or artery (2), and a second chamber (9) for housing a nozzle (3) connected at one of the ends thereof to the catheter, so that the nozzle (3) is retained inside the second chamber, and connected at the opposite end thereof to at least one external tube (4) in fluid communication with the catheter through the nozzle (3), and wherein the case (5) is flat and protrudes from the base (7) thereof over a relatively low height, and wherein the first and second chambers (8, 9) are located approximately on the same plane,
**characterized in that**
the lid (6) comprises at the internal side thereof two beads (13) entering into the second chamber (9) in the closed position of the lid (6) so as to come into abutment respectively on two lateral sides (3a) of the nozzle (3) of the catheter (1) so as to maintain it on both sides with respect to the flat bottom of the second chamber (9) also fixed to the patient's skin.

2. The device according to claim 1, **characterized in that** the two beads (13) are suitable for coming into abutment, in the closed position of the lid (6), respectively on two wings (3b) extending on either side of the lateral sides (3a) of the nozzle (3) of the catheter (1) so as to maintain the latter in abutment on the bottom of the second chamber (9).

3. The device according to claim 1 or 2, **characterized in that** the two chambers (8, 9) are separated from each other by a wall (12), the upper side of which is located in the plane of the upper side (5a) of the case (5), and comprising a communicating passage (10) defined by two slanting lateral sides (11) perpendicular to the bottom of the second chamber (9) and converging towards the first chamber (8), and **in that** the nozzle (3) of the catheter (1) has the lateral sides thereof, which extend those retained by the beads (13), of a shape matching the lateral sides (11) of the passage (10) in order to allow for the nozzle (3) of the catheter (1) to engage partly inside the passage (10) and to be retained therein.

4. The device according to any of claims 1 to 3, **characterized in that** the first chamber (8) has the bottom thereof made by a relatively thin flexible membrane (17) suitable for being fixed to the patient's skin and comprising an orifice (18) allowing for the passage of the nozzle of the catheter (3) and of this catheter.

5. The device according to claim 4, **characterized in that** the membrane (17) comprises slits (19) starting from the edge limiting the above-mentioned through-orifice (18).

6. The device according to any of claims 1 to 3, **characterized in that** the lid (6) comprises at the internal side thereof two further beads (14) entering into the first chamber (8) in the closed position of the lid (6) so as to be arranged on either side of the external part (1a) of the catheter (1) placed inside the vein (2) by largely sealing this chamber and to come in abutment on the patient's skin.

7. The device according to claim 6, **characterized in that** each bead (14) comprises on the side (14a) thereof to be in touch with the skin a colloid suitable for containing an antiseptic or antimicrobial agent.

8. The device according to any of claims 1 to 7, **characterized in that** the case (5) comprises on the upper side (5a) thereof, limiting the second chamber (9), opposite the communication (10) between both chambers (8, 9), at least one longitudinal groove (15) for receiving the external tube (4) connected to the junction nozzle (3) with the catheter (1), and the internal side of the lid (6) also comprises at least one longitudinal groove (16) to be placed on top of the groove (15) of the case (5) in the closed position of the lid (6) for holding the tube (4) with respect to the case (5), with the tube (4) leaving this case suitable for being connected to means for perfusion, transfusion, or blood collection.

9. The device according to any of claims 1, 4 to 7, **characterized in that** the second chamber (9) directly opens into the first chamber (8) and is composed by a recessed part having a shape cooperating with an identical recessed part defined between both beads (13) of the lid (6) so as to form in the closed position of the lid (6) a housing matching the nozzle (3) of the central arterial catheter (1) allowing for this nozzle to be retained inside the case (5).

10. The device according to any of the preceding claims, **characterized in that** the base (7) for fixing the case (5) is composed of a ply of flexible material integrally molded with the case (5) and, possibly the membrane (17), of the first chamber (8), with the faces of the bottom of the second chamber (8) and the membrane (17) in abutment on the patient's skin being continuous with the abutting side of the base (7) on the skin.

11. The device according to claim 10, **characterized in that** the base (7) for fixing the case (5) comprises at least two abutting tabs (7a) each being ear-shaped.

12. The device according to claim 11, **characterized in that** the base (7) comprises four ear-shaped abutting tabs (7a).

13. The device according to any of the preceding claims, **characterized in that** the lid (6) is hingedly mounted to the case (5) and can be locked to the latter by snapping.

14. The device according to any of the preceding claims, **characterized in that** the base (7) of the case (5) is suitable for being fixed to the patient's skin via a colloid suitable for containing antiseptic or antimicrobial agents.

## Patentansprüche

1. Vorrichtung zum Befestigen eines Katheters (1), wie etwa eines peripheren Venenkatheters, eines zentralen Venenkatheters oder eines zentralen Arterienkatheters, am Körper eines Patienten, umfassend ein Gehäuse (5), das durch einen Deckel (6) verschließbar ist, eine Unterlage (7), die mit dem Gehäuse (5) fest verbunden ist und dieses umgibt und es ermöglicht, das Gehäuse (5) an der Haut des Patienten zu befestigen, wobei das Gehäuse (5) zwei Kammern (8, 9) umfasst, die miteinander kommunizieren, wobei sich eine erste Kammer (8) oberhalb der Punktionsstelle des Katheters befindet und dazu geeignet ist, von dem externen Teil (1a) des in die Vene oder Arterie (2) eingesetzten Katheters (1) durchquert zu werden, und wobei eine zweite Kammer (9) die Aufnahme eines Ansatzes (3) ermöglicht, der an einem seiner Enden an den Katheter angeschlossen ist, damit der Ansatz (3) in der zweiten Kammer festgehalten wird, und an seinem gegenüberliegenden Ende an mindestens einen externen Schlauch (4) in fluidtechnischer Verbindung mit dem Katheter durch den Ansatz (3) hindurch angeschlossen ist, und wobei das Gehäuse (5) flach ist und von seiner Unterlage (7) über eine relativ geringe Höhe absteht, und wobei die ersten und zweiten Kammern (8, 9) sich ungefähr in ein und derselben Ebene befinden,
**dadurch gekennzeichnet, dass**
der Deckel (6) auf seiner Innenseite zwei Wülste (13) umfasst, die in die zweite Kammer (9) eindringen, wenn der Deckel (6) sich in der geschlossenen Position befindet, um jeweils auf zwei seitlichen Seiten (3a) des Ansatzes (3) des Katheters (1) zur Anlage zu kommen, um ihn beidseitig im Verhältnis zum flachen Boden der zweiten Kammer (9) zu halten, die ebenfalls an der Haut des Patienten befestigt ist.

2. Vorrichtung nach Anspruch 1, **dadurch gekennzeichnet, dass** die beiden Wülste (13) dazu geeignet sind, in der geschlossenen Position des Deckels (6) jeweils auf zwei Flügeln (3b) zur Anlage zu kommen, die sich auf beiden Seiten der seitlichen Seiten (3a) des Ansatzes (3) des Katheters (1) erstrecken, um diesen auf dem Boden der zweiten Kammer (9) in Anlage zu halten.

3. Vorrichtung nach Anspruch 1 oder 2, **dadurch gekennzeichnet, dass** die beiden Kammern (8, 9) voneinander durch eine Wand (12) getrennt sind, deren obere Seite sich in der Ebene der oberen Seite (5a) des Gehäuses (5) befindet und die einen Kommunikationsdurchgang (10) umfasst, der durch zwei schräge seitliche Seiten (11) definiert wird, die zum Boden der zweiten Kammer (9) rechtwinklig sind und auf die erste Kammer (8) zulaufen, und dass der Ansatz (3) des Katheters (1) seitliche Seiten in der Verlängerung derjenigen, die von den Wülsten (13) festgehalten werden, und deren Form zu den seitlichen Seiten (11) des Durchgangs (10) passt, aufweist, um es dem Ansatz (3) des Katheters (1) zu ermöglichen, teilweise in den Durchgang (10) einzugreifen und darin festgehalten zu werden.

4. Vorrichtung nach einem der Ansprüche 1 bis 3, **dadurch gekennzeichnet, dass** die erste Kammer (8) einen Boden aufweist, der durch eine biegsame und relativ dünne Membran (17) gebildet wird, die dazu geeignet ist, an der Haut des Patienten befestigt zu werden und eine Öffnung (18) umfasst, die den Durchgang des Ansatzes des Katheters (3) und dieses Katheters ermöglicht.

5. Vorrichtung nach Anspruch 4, **dadurch gekennzeichnet, dass** die Membran (17) Schlitze (19) umfasst, die vom Rand ausgehend die genannte Durchgangsöffnung (18) begrenzen.

6. Vorrichtung nach einem der Ansprüche 1 bis 3, **dadurch gekennzeichnet, dass** der Deckel (6) auf seiner Innenseite zwei weitere Wülste (14) umfasst, die in die erste Kammer (8) eindringen, wenn sich der Deckel (6) in der geschlossenen Position befindet, so dass sie auf beiden Seiten des äußeren Teils (1a) des in die Vene (2) eingesetzten Katheters (1) angeordnet sind, indem sie diese Kammer größtenteils verschließen und auf der Haut des Patienten zur Anlage kommen.

7. Vorrichtung nach Anspruch 6, **dadurch gekennzeichnet, dass** jede Wulst (14) auf ihrer Seite (14a), die dazu gedacht ist, mit der Haut in Kontakt zu kommen, ein Kolloid umfasst, das eine antiseptische oder antimikrobielle Substanz enthalten kann.

8. Vorrichtung nach einem der Ansprüche 1 bis 7, **dadurch gekennzeichnet, dass** das Gehäuse (5) auf seiner oberen Seite (5a), welche die zweite Kammer (9) begrenzt, gegenüber der Kommunikation (10) zwischen den beiden Kammern (8, 9), mindestens eine Längsrille (15) zum Aufnehmen des externen Schlauchs (4) umfasst, der an den Ansatz (3) zur Verbindung mit dem Katheter (1) angeschlossen ist, und die Innenseite des Deckels (6) ebenfalls mindestens eine Längsrille (16), die in der geschlossenen Position des Deckels (6) auf der Rille (15) des Gehäuses (5) zu liegen kommt, umfasst, um den Schlauch (4) im Verhältnis zum Gehäuse (5) zu halten, wobei der Schlauch (4), der aus diesem Gehäuse austritt, an Mittel zur Perfusion, Transfusion oder zur Blutabnahme angeschlossen werden kann.

9. Vorrichtung nach einem der Ansprüche 1, 4 bis 7, **dadurch gekennzeichnet, dass** die zweite Kammer (9) direkt in die erste Kammer (8) mündet und aus einem ausgehöhlten Teil besteht, der eine Form aufweist, die mit einem identischen ausgehöhlten Teil zusammenwirkt, der zwischen den beiden Wülsten (13) des Deckels (6) definiert ist, um in der geschlossenen Position des Deckels (6) eine zum Ansatz des zentralen Arterienkatheters (1) passende Aufnahme zu bilden, die es ermöglicht, diesen Ansatz in dem Gehäuse (5) festzuhalten.

10. Vorrichtung nach einem der vorhergehenden Ansprüche, **dadurch gekennzeichnet, dass** die Unterlage (7) zum Befestigen des Gehäuses (5) aus einer biegsamen Materiallage besteht, die mit dem Gehäuse (5) und gegebenenfalls mit der Membran (17) der ersten Kammer (8) einstückig geformt wird, wobei die Seiten des Bodens der zweiten Kammer (8) und der Membran (17), die auf der Haut des Patienten anliegen, die Anlageseite der Unterlage (7) auf der Haut fortsetzen.

11. Vorrichtung nach Anspruch 10, **dadurch gekennzeichnet, dass** die Unterlage (7) zum Befestigen des Gehäuses (5) mindestens zwei Anlagelaschen (7a) umfasst, die jeweils ohrförmig sind.

12. Vorrichtung nach Anspruch 11, **dadurch gekennzeichnet, dass** die Unterlage (7) vier ohrförmige Anlagelaschen (7a) umfasst.

13. Vorrichtung nach einem der vorhergehenden Ansprüche, **dadurch gekennzeichnet, dass** der Deckel (6) an dem Gehäuse (5) gelenkig angebracht ist und durch Einrasten mit diesem verriegelbar ist.

14. Vorrichtung nach einem der vorhergehenden Ansprüche, **dadurch gekennzeichnet, dass** die Unterlage (7) des Gehäuses (5) dazu geeignet ist, an der Haut des Patienten durch ein Kolloid befestigt zu werden, das antiseptische oder antimikrobielle Substanzen enthalten kann.
